# EUROPEAN PATENT SPECIFICATION

(11) **EP 3 960 246 B1**
(45) Date of publication and mention of the grant of the patent: **22.04.2026**
(21) Application number: 20795566.7
(22) Date of filing: 24.04.2020
(51) Int. Cl.: A61K 8/34, A61Q 19/02, A61Q 19/00, A61K 8/06, A61K 8/41, A61K 8/44, A61K 8/60, A61K 8/87, A61K 8/86, A61K 8/73, A61K 8/81

(54) **HIGH VISCOSITY O/W EMULSION COMPOSITION CONTAINING ADENOSINE PHOSPHATE**
ADENOSINPHOSPHAT-HALTIGE HOCHVISKOSE ÖL-IN-WASSER-EMULSIONSZUSAMMENSETZUNG
COMPOSITION D'ÉMULSION HUILE/EAU À VISCOSITÉ ÉLEVÉE CONTENANT DU PHOSPHATE D'ADÉNOSINE

(30) Priority: 26.04.2019 WO PCT/JP2019/018099
(43) Date of publication of application: 02.03.2022
(73) Proprietor: Otsuka Pharmaceutical Co., Ltd., Tokyo 101-8535 (JP)
(72) Inventor: OGIHARA, Miyoko, Osaka-shi, Osaka 540-0021 (JP); SUMA, Momoko, Osaka-shi, Osaka 540-0021 (JP); MIYAWAKI, Shiori, Osaka-shi, Osaka 540-0021 (JP)
(74) Representative: Hoffmann Eitle
(86) International application number: PCT/JP2020/017786
(87) International publication number: WO 2020/218547

(56) References cited:
- EP-A1- 1 512 405
- EP-A1- 2 939 657
- EP-A1- 3 095 439
- EP-A1- 3 960 245
- WO-A1-02/41853
- WO-A1-2009/148059
- WO-A1-2015/107709
- JP-A- 2002 234 830
- JP-A- 2007 169 240
- JP-A- 2009 286 757
- JP-A- 2018 016 584
- JP-A- H0 919 631

## Description

### Technical Field

The present invention is as defined in the appended claims and relates to a high viscosity O/W emulsion composition comprising an adenosine phosphate and/or a salt thereof, and an acrylic acid-alkyl methacrylate copolymer.

### Background Art

Adenosine phosphate is known to have a moisturizing effect and a whitening effect, and cosmetics containing adenosine phosphate have already been studied in various ways (for example, Patent Document 1), and have been put on the market.

Acrylic acid-alkyl methacrylate copolymers are known as polymeric emulsifiers suitable for the formulation of gel creams and emulsions having a lightly and fresh feeling during use.

Patent Document 2 describes compositions for improving melasma and reducing skin dullness comprising a purine nucleic acid-related substance and a pharmaceutically or cosmetically acceptable carrier.

Patent Document 3 relates to a skin emulsion composition having a further improved thickening effect which has a viscosity of 10,000 mPa·s or more and comprises at least one member selected from the group consisting of polyacrylic acids and salts thereof, an alkyl-modified carboxy polymer, and an alkanolamine compound.

Patent Document 4 describes O/W-type emulsion compositions having a favorable emulsion stability and containing an electrolyte, a polyglyceryl fatty acid ester, alkanoyl lactic acid of its salt, an acrylic acid alkyl methacrylate copolymer, a water/moisture content and an oil component.

Patent Document 5 relates to the provision of a low viscosity O/W emulsion composition having a good emulsion stability, comprising an adenosine phosphate and/or a salt thereof; an acrylic acid-alkyl methacrylate copolymer; an oil content; and water; and comprising a water-soluble polymer selected from a swelling thickener, an associative thickener, a polysaccharide and a derivative thereof and a mixture thereof, and/or a pH adjuster which is an organic alkali compound; wherein the composition has a viscosity of less than 10,000 mPa·s.

Patent Document 6 concerns an external dermal composition for anti-ageing, which prevents or improves apparent skin problems such as wrinkles, fine wrinkles, blemishes, and saggings caused by increasing age or ageing; and maintains or improves the barrier function and the hyaluronic acid production in the skin and contains an aqueous medium as a base material and one or more ingredients selected from L-ascorbic acid, derivatives thereof, and their salts as an effective ingredient(s).

### Prior Art Document

### Patent Document

[Patent Document 1] WO 2002/41853
[Patent Document 2] EP 1 512 405 A1
[Patent Document 3] EP 3 095 439 Al
[Patent Document 4] JP 2002 234830 A
[Patent Document 5] EP 3 960 245 Al
[Patent Document 6] EP 2 939 657 Al

### Summary

### Technical Problem

The present inventors attempted to develop an O/W emulsion composition containing an adenosine phosphate and an acrylic acid-alkyl methacrylate copolymer and having a high viscosity (equal to or higher than 10,000 mPa·s), but encountered the problem of not being able to obtain a composition having a good emulsification stability. The object of the present invention is to provide a high viscosity (equal to or higher than 10,000 mPa·s) O/W emulsion composition comprising an adenosine phosphate and/or a salt thereof and an acrylic acid-alkyl methacrylate copolymer and having a good emulsification stability.

### Solution to Problem

The present inventors have found that unexpectedly, a specific water-soluble polymer and a specific pH adjuster respectively improve the emulsification stability of a composition comprising an adenosine phosphate and/or a salt thereof and an acrylic acid-alkyl methacrylate copolymer.

In addition, the present inventors have found that an addition of an uncrystallized high melting point oil having a melting point of 30 °C or higher; an oil-based gelling agent; and/or a silicone agent for improving feel further improve the feel during use of the composition.

The present invention is as defined in the appended claims.
Specifically, the present application provides the following aspects of the invention.

An O/W emulsion composition,
comprising Ingredient (A), Ingredient (B), Ingredient (E), and Ingredient (F); and
comprising Ingredient (C) and/or Ingredient (D);
wherein

| | |
|---|---|
| Ingredient (A): | an adenosine phosphate and/or a salt thereof; |
| Ingredient (B): | an acrylic acid-alkyl methacrylate copolymer; |
| Ingredient (C): | a water-soluble polymer which is a urethane associativethickener; |
| Ingredient (D): | a pH adjuster which is an organic alkali compound selected from tromethamine, aminomethyl propanol, arginine,and a mixture thereof; |
| Ingredient (E): | an oil content; |
| Ingredient (F): | water, and |

wherein the composition has a viscosity of equal to or higher than 10,000 mPa·s (for example, 10,000 to 200,000 mPa·s, 15,000 to 150,000 mPa·s, 20,000 to 100,000 mPa·s, 20,000 to 40,000 mPa·s).

Preferably, Ingredient (B) is an acrylic acid-alkyl methacrylate copolymer having a molecular weight of 100,000 to 5,000,000.

Preferably, Ingredient (B) is an acrylic acid-alkyl methacrylate copolymer wherein the alkyl group has 8 to 35 carbon atoms.

More preferably, Ingredient (B) is acrylates/C10-30 alkyl acrylate crosspolymer.

Preferably, Ingredient (C) is a water-soluble polymer which is a urethane associative thickener selected from the group consisting of PEG-240/HDI copolymer bis-decyltetradeceth-20 ether, PEG-150/stearyl alcohol/SMDI copolymer, PEG-150/decyl alcohol/SMDI copolymer, polyurethane-59 and a mixture thereof.

More preferably, Ingredient (C) is PEG-240/HDI copolymer bis-decyltetradeceth-20 ether.

Preferably, Ingredient (D) is a pH adjuster which is an organic alkali compound selected from tromethamine, arginine and a mixture thereof.

More preferably, Ingredient (D) is tromethamine.

Preferably, the composition comprises Ingredient (C), and Ingredient (C)/Ingredient (A) (weight ratio) is 0.01 to 50 (preferably 0.05 to 30, more preferably 0.1 to 20, more preferably 0.2 to 10, 0.2 to 5, or even more preferably 0.3 to 0.8).

Preferably, the composition comprises Ingredient (C), and Ingredient (C)/(Ingredient (A) + Ingredient (B)) (weight ratio) is 0.01 to 50 (preferably 0.01 to 30, more preferably 0.02 to 20, more preferably 0.1 to 10, even more preferably 0.2 to 3, or even more preferably 0.2 to 0.7).

Preferably, the composition has a pH of 5.5 to 7.5 (preferably pH 6 to 7, pH 6.5 to 7).

Preferably, the composition comprises Ingredient (D) and has a pH of 5.5 to 7.5 (preferably pH 6 to 7, pH 6.5 to 7).

Preferably, the composition does not comprise a pH adjuster which is an inorganic alkali compound, or
the composition comprises a pH adjuster which is an inorganic alkali compound, and the pH adjuster which is an inorganic alkali compound/Ingredient (D) (weight ratio) is 0.1 or less (preferably 0.01 or less).

Preferably, Ingredient (C)/Ingredient (B) (weight ratio) is 0.001 to 200 (preferably 0.01 to 150, more preferably 0.03 to 100, even more preferably 0.2 to 10, even more preferably 0.8 to 4, or even more preferably 0.75 to 3.75).

Preferably, the composition comprises 0.001 to 10% by weight (preferably 0.01 to 10% by weight, more preferably 0.1 to 7% by weight, more preferably 0.2 to 5% by weight, even more preferably 0.3 to 4% by weight, still more preferably 0.4% to 3.5% by weight, more preferably 1 to 3% by weight) of Ingredient (A).

Preferably, the composition comprises 0.05 to 5% by weight (preferably 0.1 to 4% by weight, more preferably 0.15 to 3% by weight, even more preferably 0.2 to 2% by weight, still more preferably 0.4 to 0.8% by weight) of Ingredient (B).

Preferably, the composition comprises 0.01 to 20% by weight (0.01 to 15% by weight, more preferably 0.1 to 10% by weight, more preferably 0.3 to 5% by weight, even more preferably 0.5 to 2% by weight, even more preferably 0.6% by weight to 1.5% by weight) of Ingredient (C).

Preferably, the composition comprises 1 to 60% by weight (preferably 3 to 50% by weight, more preferably 5 to 40% by weight, more preferably 5 to 30% by weight, even more preferably 5% to 20% by weight, still more preferably 5% by weight to 16% by weight) of Ingredient (E).

Preferably, the composition comprises 20 to 99% by weight (preferably 30 to 99% by weight, more preferably 40 to 99% by weight, more preferably 50 to 99% by weight, even more preferably 60 to 95% by weight, still more preferably 65 to 90% by weight) of Ingredient (F).

Preferably, Ingredient (E) comprises Ingredient (G) selected from an uncrystallized high melting point oil having a melting point of 30 °C or higher; an oil-based gelling agent; a silicone agent for improving feel; and a mixture thereof.

Preferably, Ingredient (G) is selected from:
an uncrystallized high melting point oil having a melting point of 30 °C or higher selected from an uncrystallized triglyceride having a melting point of 30 °C or higher; an uncrystallized sterol fatty acid ester having a melting point of 30 °C or higher; petrolatum; and a mixture thereof;
an oil-based gelling agent selected from an inulin fatty acid ester, a dextrin fatty acid ester, polyglycerol, dibutyl ethylhexanoyl glutamide, dibutyl lauroyl glutamide, sodium dilauramidoglutamide lysine, vinyl dimethicone/methicone silsesquioxane crosspolymer, PEG/PPG-19/19 dimethicone, and a mixture thereof;
a silicone agent for improving feel selected from an amino-modified silicone, a silicone wax, polyether-modified silicone, highly polymerized dimethicone, a silicone wax, a silicone emulsion, a silicone powder, and a mixture thereof; and
a mixture thereof.

Preferably, Ingredient (G) is selected from caprylic/capric/myristic/stearic triglyceride, phytosteryl/behenyl/octyldodecyl lauroyl glutamate, an inulin fatty acid ester (for example, stearoyl inulin), a dextrin fatty acid ester (for example, dextrin myristate), highly polymerized dimethicone, stearyl dimethicone, and a mixture thereof.

Preferably, Ingredient (G) is selected from caprylic/capric/myristic/stearic triglyceride, phytosteryl/behenyl/octyldodecyl lauroyl glutamate, and a mixture thereof.

Preferably, Ingredient (G) is selected from an inulin fatty acid ester (for example, stearoyl inulin), a dextrin fatty acid ester (for example, dextrin myristate), and a mixture thereof.

Preferably, Ingredient (G) is selected from highly polymerized dimethicone, stearyl dimethicone, and a mixture thereof.

Preferably, the amount of Ingredient (G) is 0.1 to 50% by weight (preferably 0.5 to 30% by weight, more preferably 1 to 20% by weight, still more preferably 2 to 13% by weight) based on Ingredient (E).

Preferably, Ingredient (G) is an uncrystallized high melting point oil having a melting point of 30 °C or higher, and the amount of Ingredient (G) is 0.1 to 30% by weight (preferably 0.1 to 20% by weight, more preferably 0.5 to 15% by weight, still more preferably 3 to 10% by weight) based on Ingredient (E).

Preferably, Ingredient (G) is an oil-based gelling agent, and the amount of Ingredient (G) is 0.01 to 30% by weight (preferably 0.1 to 20% by weight, more preferably 0.5 to 10% by weight, still more preferably 2 to 7% by weight) based on Ingredient (E).

Preferably, Ingredient (G) is a silicone agent for improving feel, and the amount of Ingredient (G) is 0.01 to 50% by weight (preferably 0.1 to 30% by weight, more preferably 0.1 to 20% by weight, still more preferably 1 to 20% by weight, still even more preferably 3 to 15% by weight) based on Ingredient (E).

Preferably, the composition comprises 0.001 to 20% by weight (preferably 0.01 to 10% by weight, more preferably 0.1 to 7% by weight, more preferably 0.5 to 5% by weight, even more preferably 0.7 to 3% by weight, still more preferably 0.3 to 2% by weight) of Ingredient (G).

Preferably, the composition further comprises Ingredient (H): a water-soluble humectant.

Preferably, Ingredient (H) is a water-soluble humectant selected from a low-polarity polyhydric alcohol (for example, butylene glycol, bis-ethoxydiglycol cyclohexane 1,4-dicarboxylate, dipropylene glycol, PEG/PPG/polybutylene glycol-8/5/3 glycerin, POE methylglucoside), a saccharide (for example, glyceryl glucoside, diglycerin, trehalose, a sorbitol culture polysaccharide solution), a film forming agent (for example, pullulan, PVP, gum arabic), and a mixture thereof.

In a preferred embodiment, the O/W emulsion composition comprises

| | |
|---|---|
| Ingredient (A): | an adenosine phosphate and/or a salt thereof; |
| Ingredient (B): | acrylates/C10-30 alkyl acrylate crosspolymer; |
| Ingredient (C): | PEG-240/HDI copolymer bis-decyltetradeceth-20ether; |
| Ingredient (E): | an oil content; and |
| Ingredient (F): | water, |

wherein the composition has a viscosity of 20,000 to 40,000 mPa·s, and wherein Ingredient (C)/(Ingredient (A) + Ingredient (B)) (weight ratio) preferably is 0.2 to 0.7.

In a further preferred embodiment, the O/W emulsion composition comprises

| | |
|---|---|
| Ingredient (A): | an adenosine phosphate and/or a salt thereof1.5 to 3.5% by weight; |
| Ingredient (B): | acrylates/C10-30 alkyl acrylate crosspolymer0.4 to 0.8% by weight; |
| Ingredient (C): | PEG-240/HDI copolymer bis-decyltetradeceth-20ether 0.3 to 0.8% by weight; |
| Ingredient (E): | an oil content 5 to 20% by weight;and |
| Ingredient (F): | water, |

wherein the composition has a viscosity of 20,000 to 40,000 mPa·s.

In another preferred embodiment, the O/W emulsion composition comprises

| | |
|---|---|
| Ingredient (A): | an adenosine phosphate and/or a salt thereof; |
| Ingredient (B): | acrylates/C10-30 alkyl acrylate crosspolymer; |
| Ingredient (D): | tromethamine and/or arginine; |
| Ingredient (E): | an oil content; and |
| Ingredient (F): | water, |

wherein the composition has a viscosity of 20,000 to 40,000 mPa·s, and a pH of 6 to 7.

[40] In a further preferred embodiment, the O/W emulsion comprises

| | |
|---|---|
| Ingredient (A): | an adenosine phosphate and/or a salt thereof1.5 to 3.5% by weight; |
| Ingredient (B): | acrylates/C10-30 alkyl acrylate crosspolymer0.4 to 0.8% by weight; |
| Ingredient (D): | tromethamine, and/or arginine; |
| Ingredient (E): | an oil content 5 to 20% by weight;and |
| Ingredient (F): | water, |

wherein the composition has a viscosity of 20,000 to 40,000 mPa·s, and a pH of 6 to 7.

Preferably, the O/W emulsion composition is a lotion; an emulsion selected from an emollient emulsion, a milky lotion, a nourishing emulsion, and an cleansing emulsion; a cream selected from an emollient cream, a massage cream, a cleansing cream, and a makeup cream; or a lip balm.

Preferably, the O/W emulsion composition is applied to or sprayed on skin.

Also disclosed herein are the following aspects:
A method for producing an O/W emulsion composition having a viscosity of equal to or higher than 10,000 mPa·s, comprising mixing Ingredient (A), Ingredient (B), Ingredient (E), and Ingredient (F) in the presence of Ingredient (C) and/or Ingredient (D),
wherein

| | |
|---|---|
| Ingredient (A): | an adenosine phosphate and/or a salt thereof; |
| Ingredient (B): | an acrylic acid-alkyl methacrylate copolymer; |
| Ingredient (C): | a water-soluble polymer selected from a swelling thickener, an associative thickener, a polysaccharide anda derivative thereof, and a mixture thereof; |
| Ingredient (D): | a pH adjuster which is an organic alkali compound; |
| Ingredient (E): | an oil content; |
| Ingredient (F): | water. |

A method for improving an emulsification stability of an O/W emulsion composition comprising Ingredient (A), Ingredient (B), Ingredient (E), and Ingredient (F) and having a viscosity of equal to or higher than 10,000 mPa·s,
comprising adding Ingredient (C) and/or Ingredient (D),
wherein

| | |
|---|---|
| Ingredient (A): | an adenosine phosphate and/or a salt thereof; |
| Ingredient (B): | an acrylic acid-alkyl methacrylate copolymer; |
| Ingredient (C): | a water-soluble polymer selected from a swelling thickener, an associative thickener, a polysaccharide anda derivative thereof, and a mixture thereof; |
| Ingredient (D): | a pH adjuster which is an organic alkali compound; |
| Ingredient (E): | an oil content; |
| Ingredient (F): | water. |

A method for improving a feel during use of an O/W emulsion composition comprising Ingredient (A), Ingredient (B), Ingredient (E), and Ingredient (F) and having a viscosity of equal to or higher than 10,000 mPa·s,
comprising adding Ingredient (C) and/or Ingredient (D),
wherein

| | |
|---|---|
| Ingredient (A): | an adenosine phosphate and/or a salt thereof; |
| Ingredient (B): | an acrylic acid-alkyl methacrylate copolymer; |
| Ingredient (C): | a water-soluble polymer selected from a swelling thickener, an associative thickener, a polysaccharide and a derivative thereof, and a mixture thereof; |
| Ingredient (D): | a pH adjuster which is an organic alkali compound; |
| Ingredient (E): | an oil content; |
| Ingredient (F): | water. |

### Advantageous Effects of Invention

The present invention provides a high viscosity (equal to or higher than 10,000 mPa·s) O/W emulsion composition comprising an adenosine phosphate and/or a salt thereof and an acrylic acid-alkyl methacrylate copolymer and having a good emulsification stability. In addition, the feel of the composition during use is improved by adding an uncrystallized high melting point oil having a melting point of 30 °C or higher, an oil-based gelling agent, and/or a silicone agent for improving feel.

### BRIEF DESCRIPTION OF THE DRAWINGS

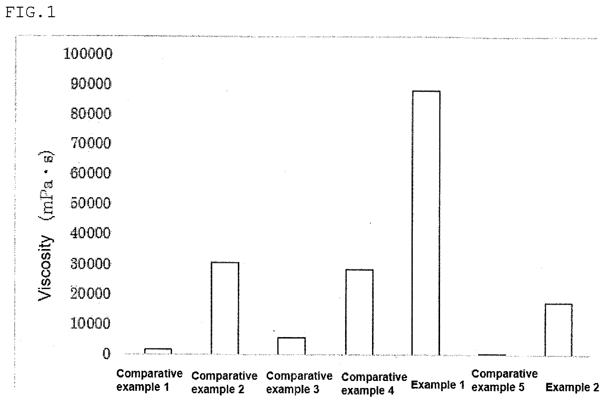

[FIG. 1] FIG. 1 shows the viscosity results of the gels before emulsification of Test Example 1.

In one aspect, the present disclosure provides an O/W emulsion composition,
comprising Ingredient (A), Ingredient (B), Ingredient (E), and Ingredient (F), and
comprising Ingredient (C) and/or Ingredient (D), and
wherein

| | |
|---|---|
| Ingredient (A): | an adenosine phosphate and/or a salt thereof; |
| Ingredient (B): | an acrylic acid-alkyl methacrylate copolymer; |
| Ingredient (C): | a water-soluble polymer which is a urethane associative thickener; |
| Ingredient (D): | a pH adjuster which is an organic alkali compound selected from tromethamine, aminomethyl propanol, arginine, and a mixture thereof; |
| Ingredient (E): | an oil content; |
| Ingredient (F): | water, and |

wherein the composition has a viscosity of equal to or higher than 10,000 mPa·s (hereinafter sometimes referred to as "the composition of the present invention").

The emulsification stability and/or the feel during use of the high viscosity O/W emulsion composition comprising an adenosine phosphate and/or a salt thereof and an acrylic acid-alkyl methacrylate copolymer may be improved by adding Ingredient (C) and/or Ingredient (D).

As used herein, the term "O/W emulsion composition" means an emulsion composition in which oil is dispersed in an aqueous phase. The method for determine whether a emulsion composition is O/W type is not particularly limited, and can be tested by a commonly used method (for example, dispersion, electrical conduction, dye, dilution, and refractive index methods).

In one embodiment, the viscosity of the composition of the present invention is higher than 10,000 mPa·s. The preferred viscosity range of the present composition may vary depending on the use, e.g., form, but may include 10,000 to 200,000 mPa·s, 15,000 to 150,000 mPa·s, 20,000 to 100,000 mPa·s, or 20,000 to 40,000 mPa·s. Further, examples of the lower limit of the viscosity of the composition of the present invention include 10,000 mPa·s, 15,000 mPa·s, 20,000 mPa·s, 30,000 mPa·s. Examples of the upper limit include 200,000 mPa·s, 150,000 mPa·s, 100,000 mPa·s, 90,000 mPa·s, 80,000 mPa·s, 70,000 mPa·s, 60,000 mPa·s. Preferred examples of the range may be shown by a combination of the lower limit and the upper limit.

In the present disclosure, a viscosity of a composition is determined by a single cylinder-type rotational viscometer (B-type viscometer, Tokyo Keiki BM type), rotor No. 4 at 25 °C, 12 rpm or 30 rpm for 1 minute. Alternatively, a viscosity of a composition is determined by a single cylinder-type rotational viscometer (B-type viscometer, Tokyo Keiki BH type), rotor No.7 at 25 °C, 6 to 10 rpm for 1 minute.

Alternatively, it may be determined in a manner equivalent to those described above.

In the present application, examples of an adenosine phosphate include adenosine monophosphate (e.g., adenosine 2'-monophosphate, adenosine 3'-monophosphate, adenosine 5'-monophosphate), adenosine diphosphate (e.g., adenosine 5'-diphosphate), adenosine triphosphate (e.g., adenosine 5'-triphosphate), and adenosine 3',5'-cyclic phosphate, and a single kind of the adenosine phosphate may be used, or any combination of two or more kinds of the adenosine phosphate may be used.

In the present application, a salt of adenosine phosphate(s) is not particularly limited as long as it can be formulated in a cosmetic, a drug or quasi-drug for external use. Examples of salts of adenosine monophosphate include specifically alkali metal salts such as sodium salts and potassium salts; alkaline earth metal salts such as calcium salts, magnesium salts and barium salts; basic amino acid salts such as arginine and lysine; ammonium salts such as ammonium salts and tricyclohexylammonium salts; alkanolamine salts such as monoethanolamine salts, diethanolamine salts, triethanolamine salts, monoisopropanolamine salts, diisopropanolamine salts and triisopropanolamine, and a single kind of the salt of adenosine phosphate(s) may be used, or any combination of two or more kinds of the salt of adenosine phosphate(s) may be used.

The amount of Ingredient (A) comprised in the composition of the present invention may vary depending on the use, e.g., form, of the composition, but may be optionally selected from a range of, for example, usually 0.001 to 10% by weight based on the total weight of the composition. Preferably 0.01% by weight to 10% by weight, more preferably 0.1% by weight to 7% by weight, more preferably 0.2% by weight to 5% by weight, even more preferably 0.3% by weight to 4% by weight, still more preferably 0.4% by weight to 3.5% by weight, more preferably 1% by weight to 3% by weight are exemplified.

Further examples of the lower limit of the amount range of Ingredient (A) comprised in the composition of the present invention include 0.001% by weight, 0.01% by weight, 0.1% by weight, 0.2% by weight, 0.3% by weight, 0.4% by weight, 0.5% by weight, 0.7% by weight, and 1% by weight, and examples of the upper limit include 2% by weight, 3% by weight, 3.5% by weight, 4% by weight, 5% by weight, 7% by weight, and 10% by weight, and preferred examples of the range may be shown by a combination of the lower limit and the upper limit.

In the present application, an acrylic acid-alkyl methacrylate copolymer is not particularly limited, and for example, an acrylic acid-alkyl methacrylate copolymer having a molecular weight of 100,000 to 5,000,000 is exemplified. Examples of an acrylic acid-alkyl methacrylate copolymer include an acrylic acid-alkyl methacrylate copolymer wherein the alkyl group has 8 to 35 carbon atoms, more preferably an acrylic acid-alkyl methacrylate copolymer wherein the alkyl group has 10 to 30 carbon atoms (for example, acrylates/C10-30 alkyl acrylate crosspolymer). The structure thereof is not limited, and are appropriately selected according to a purpose. A single kind of the acrylic acid-alkyl methacrylate copolymer may be used, or any combination of two or more kinds of the acrylic acid-alkyl methacrylate copolymer may be used in the composition of the present invention.

The amount of Ingredient (B) comprised in the composition of the present invention may vary depending on the use, e.g., form, of the composition, but may be optionally selected from, for example, a range of 0.05 to 5% by weight based on the total weight of the composition. More preferably 0.1% by weight to 4% by weight, more preferably 0.15% by weight to 3% by weight, even more preferably 0.2% by weight to 2% by weight, still more preferably 0.4% by weight to 0.8% by weight are exemplified.

Further, examples of the lower limit of the amount range of Ingredient (B) comprised in the composition of the present invention include 0.05, 0.1% by weight, 0.15% by weight, 0.2% by weight, 0.3% by weight, 0.4% by weight, and examples of the upper limit include 0.4% by weight, 0.6% by weight, 0.7% by weight, 0.8% by weight, 1% by weight, 1.5% by weight, 2% by weight, 3% by weight, 4% by weight, 5% by weight, and preferred examples of the range may be shown by a combination of the lower limit and the upper limit.

Examples of a water-soluble polymer which may be comprised in the composition of the present invention include a swelling thickener, an associative thickener, a polysaccharide and a derivative thereof. A single kind of the water-soluble polymer may be used, or any combination of two or more kinds of the water-soluble polymer may be used in the composition of the present invention.

In one embodiment, the O/W emulsion composition comprises a water-soluble polymer which is a urethane associative thickener as Ingredient (C).

Examples of a swelling thickener include an alkali swelling thickener such as sodium polyacrylate, ammonium polyacrylate, acrylamide/sodium acrylate copolymer, vinylpyrrolidone/2-acrylamide-2-methylpropanesulfonic acid copolymer, a mixture of polyacrylamide and sodium polyacrylate, sodium acrylate/2-acrylamide-2-methylpropanesulfonic acid copolymer, hydroxyethyl acrylate/2-acrylamide-2-methylpropanesulfonic acid copolymer. A single kind of the swelling thickener may be used, or any combination of two or more kinds of the swelling thickener may be used in the composition of the present invention.

Examples of an associative thickener include a polyacrylic acid-based associative thickener: for example, acrylic acid copolymer, acrylates/vinyl neodecanoate crosspolymer, acrylates/steareth-20 methacrylate crosspolymer and acrylates/beheneth-25 methacrylate copolymer; polyvinyl alcohol; a polyvinyl-based thickener; a polyether-based associative thickener; a polyglycol-based associative thickener; a maleic anhydride copolymer-based associative thickener; a polyamide-based associative thickener; a polyester-based associative thickener; a hydrophobic cellulose ester-based associative thickener; a urethane associative thickener: for example, PEG-240/HDI copolymer bis-decyltetradeceth-20 ether, PEG-150/stearyl alcohol/SMDI copolymer, PEG-150/decyl alcohol/SMDI copolymer and polyurethane-59; a polycarboxylic acid-based associative thickener, and a single kind of the associative thickener may be used, or any combination of two or more kinds of the associative thickener may be used.

Examples of a polysaccharide and a derivative thereof include cationic xanthan gum, cationic cellulose, xanthan gum, guar gum, carrageenan, tamarind gum, quince seed gum, sclerotium gum, and agar. A single kind of the polysaccharide and a derivative thereof may be used, or any combination of two or more kinds of the polysaccharide and a derivative thereof may be used.

In the composition of the present invention, the amount of Ingredient (C) is not particularly limited so long as an emulsifying property of the composition is stable and/or the composition exhibits a good feel during use, but the weight ratio of Ingredient (C) to Ingredient (A) (Ingredient (C)/Ingredient (A)) is, for example, 0.01 to 50, preferably 0.05 to 30, more preferably 0.1 to 20, more preferably 0.2 to 10, 0.2 to 5, still even more preferably 0.3 to 0.8. Further, examples of the lower limit of the (Ingredient (C)/Ingredient (A)) (weight ratio) include 0.01, 0.02, 0.05, 0.1, 0.2, 0.3, and examples of the upper limit include 0.8, 1, 3, 5, 10, 20, 30, 50, and preferred examples of the range may be shown by a combination of the lower limit and the upper limit.

Regarding the amount of Ingredient (C) in the composition of the present invention, for example, the weight ratio of Ingredient (C) to the sum of Ingredient (A) and Ingredient (B) (Ingredient (C)/(Ingredient (A) + Ingredient (B)) is 0.01 to 50, preferably 0.01 to 30, more preferably 0.02 to 20, more preferably 0.1 to 10, even more preferably 0.2 to 3, still even more preferably 0.2 to 0.7.

Further, examples of the lower limit of the (Ingredient (C)/(Ingredient (A) + Ingredient (B)) (weight ratio) include 0.01, 0.02, 0.1 and 0.2, and examples of the upper limit include 50, 30, 20, 10, 5, 3, 2, 1, 0.7 and 0.6, and preferred examples of the range may be shown by a combination of the lower limit and the upper limit.

In the composition of the present invention, the amount of Ingredient (C) is not particularly limited so long as an emulsifying property of the composition is stable and/or the composition exhibits a good feel during use, but the weight ratio of Ingredient (C) to Ingredient (B) (Ingredient (C)/Ingredient (B)) is, for example, 0.001 to 200, preferably 0.01 to 150, more preferably 0.03 to 100, even more preferably 0.2 to 10, even more preferably 0.8 to 4, or even more preferably 0.75 to 3.75. Further, examples of the lower limit of (Ingredient (C)/Ingredient (B)) (weight ratio) include 0.001, 0.01, 0.03, 0.1, 0.2, 0.3, 0.5 and 0.75, and examples of the upper limit include 200, 150, 100, 50, 30, 15, 10, 5 and 3.75, and preferred examples of the range may be shown by a combination of the lower limit and the upper limit.

The amount of Ingredient (C) comprised in the composition of the present invention may be optionally selected from, for example, a range of usually 0.01 to 20% by weight based on the total weight of the composition. Preferably 0.01 to 15% by weight, more preferably 0.1 to 10% by weight, more preferably 0.3 to 5% by weight, even more preferably 0.5 to 2% by weight, even more preferably 0.6 to 1.5% by weight are exemplified.

Further, examples of the lower limit of the amount range of Ingredient (C) comprised in the composition of the present invention include 0.01% by weight, 0.1% by weight, 0.3% by weight, 0.6% by weight, and examples of the upper limit include 20% by weight, 10% by weight, 5% by weight, 3% by weight, 1.5% by weight, and preferred examples of the range may be shown by a combination of the lower limit and the upper limit.

Examples of a pH adjuster which is an organic alkali compound include tromethamine, aminomethyl propanediol, aminomethyl propanol, and arginine, and a single kind of the pH adjuster may be used, or any combination of two or more kinds of the pH adjuster may be used.

In one embodiment, the O/W emulsion composition comprises a pH adjuster which is an organic alkali compound selected from tromethamine, aminomethyl propanol, arginine, and a mixture thereof as Ingredient (D).

The preferred pH of the composition of the present invention varies depending on the useof the composition, and includes, for example, pH 5.5 to 7.5 (preferably pH 6 to 7, such as pH 6.5 to 7). In adjusting the pH of the composition of the present invention, a pH adjuster (for example, a pH adjuster of an inorganic alkali compound (for example, potassium hydroxide, sodium hydroxide))) other than Ingredient (D) may be used to the extent that the effect on the emulsification stability and/or the feeling during use is not problematic. When Ingredient (D) and an inorganic alkali compound are used together, in the composition of the present invention, the weight ratio of the pH adjuster of inorganic alkali compound to Ingredient (D) (a pH adjuster of an inorganic alkali compound /Ingredient (D)) is preferably 0.1 or less (more preferably 0.01 or less). In one embodiment, an organic alkali compound may be used in the composition of the present invention in view of its ability to inhibit discoloration with time and/or change of odour with time of the composition.

In the composition of the present invention, the amount of Ingredient (D) is not particularly limited as long as the emulsification stability and/or the feel during use of the composition is good, and varies depending on the amount of Ingredient (A), the type and amount of other ingredients and the preferred pH of the composition. For example, the weight ratio of Ingredient (D) to Ingredient (A) (Ingredient (D)/Ingredient (A)) is 0.01 to 100 (for example, 0.05 to 50, 0.1 to 30). Further examples of the lower limit of the "Ingredient (D)/Ingredient (A) (weight ratio)" include 0.01, 0.05 and 0.1, and examples of the upper limit include 1, 5, 10, 20, 30, 50, 70 and 100, and preferred examples of the range may be shown by a combination of the lower limit and the upper limit.

In the composition of the present invention, the oil content of Ingredient (E) is not particularly limited.

Specifical examples of the oil content includes
oils, for example vegetable oils such as peanut oil, sesame oil, soybean oil, safflower oil, avocado oil, sunflower oil, corn oil, rapeseed oil, cottonseed oil, castor oil, camellia oil, coconut oil, olive oil, poppy oil, cacao oil or jojoba oil, and animal oils such as beef tallow, pork tallow or wool oil;
hydrocarbon-based liquid oils such as petrolatum, liquid paraffin, squalane, hydrogenated polyisobutene or α-olefin oligomer;
higher fatty acid esters such as isopropyl myristate, isopropyl isostearate, myristyl myristate, cetyl ethylhexanoate, cetyl palmitate, cetyl isooctanoate, isocetyl myristate, n-butyl myristate, octyldodecyl myristate, isopropyl linolenate, propyl ricinoleate, isopropyl ricinoleate, pentaerythritol tetraoctanoate, isobutyl ricinoleate, heptyl ricinoleate, diethyl sebacateor diisopropyl adipate; sterol fatty acid esters such as phytosteryl isostearate or phytosteryl/octyldodecyl lauroyl glutamate; lanolins and derivatives thereof such as lanolin, lanolin oil or lanolin wax; waxes such as bleached beeswax, spermaceti or Japan wax;
higher aliphatic alcohols such as cetyl alcohol, stearyl alcohol, behenyl alcohol, batyl alcohol or chimyl alcohol;
Waxes;
higher fatty acids such as stearic acid, oleic acid or palmitic acid;
mixtures of mono-, di-, tri-glyceride of saturated or unsaturated fatty acids having 12 to 18 carbon atoms;
silicone oils, for example, chain silicones such as methyl polysiloxane, dimethylpolysiloxane, methylphenylpolysiloxane or methylhydrogenpolysiloxane, , cyclic silicones such as cyclopentasiloxane, decamethylcyclopentasiloxane, octamethylcyclotetrasiloxane or methylcyclosiloxane, crosslinked silicones such as crosslinked methylpolysiloxane or crosslinked methylphenylpolysiloxane, modified silicones modified, for example, with polyoxyethylene or polyoxypropylene.

The amount of Ingredient (E) comprised in the composition of the present invention may be optionally selected from a range of, for example, usually 1 to 60% by weight based on the total weight of the composition. Preferably 3 to 50% by weight, more preferably 5 to 40% by weight, more preferably 5 to 30% by weight, even more preferably 5 to 20% by weight, still more preferably 5 to 16% by weight are exemplified. Further examples of the lower limit of the amount range of Ingredient (E) comprised in the composition of the present invention include 1% by weight, 3% by weight, 5% by weight, 8% by weight, 10% by weight, 15% by weight, and examples of the upper limit include 60% by weight, 50% by weight, 40% by weight, 30% by weight, 20% by weight, 16% by weight, and preferred examples of the range may be shown by a combination of the lower limit and the upper limit.

In the composition of the present invention, Ingredient (E) may comprise Ingredient (G) selected from an uncrystallized high melting point oil having a melting point of 30 °C or higher; an oil-based gelling agent; a silicone agent for improving feel; and a mixture thereof. The feel during use of the composition of the present invention may be further improved by adding Ingredient (G) to the composition of the present invention.

Regarding the feel during use of the composition of the present invention, an addition of an uncrystallized high melting point oil having a melting point of 30 °C or higher into the composition of the present invention may improve the thickness feeling and the richness feeling while unexpectedly reducing the stickiness feeling and improving the fresh feeling. Although it is generally difficult to obtain a stable emulsification when adding a high melting point oil into an emulsion composition comprising a polymeric emulsifier, it may be easy to obtain a stable emulsification by using an uncrystallized high melting point oil in the emulsion composition.

Examples of an uncrystallized high melting point oil include
an uncrystallized triglyceride having a melting point of 30 °C or higher: for example, caprylic/capric/myristic/stearic triglyceride, theobroma grandiflorum seed butter, shea butter, hydrogenated palm oil, hydrogenated jojoba oil, jojoba butter, cocoa butter, palm oil, palm kernel oil, hydrogenated oil, coconut acid, PEG-60 almond glycerides, irvingia gabonensis kernel butter, hydrogenated coco-glycerides, lanolin acid;
petrolatum;
an uncrystallized sterol fatty acid ester having a melting point of 30 °C or higher: for example, phytosteryl macadamiate, polyoxyethylene phytosterol (5E.O.), phytosteryl sunflowerseedate, phytosteryl/behenyl/octyldodecyl lauroyl glutamate, bis-behenyl/isostearyl/phytosteryl dimer dilinoleyl dimer dilinoleate, phytosteryl/isostearyl/cetyl/stearyl/behenyl dimer dilinoleate, raspberry seed oil/tocopheryl succinate aminopropanediol esters, diglyceryl adipate mixed fatty acid ester, bis-diglyceryl polyacyladipate-2).

A single kind of the uncrystallizede high melting point oil may be used, or any combination of two or more kinds of the uncrystallized high melting point oil may be used.

Regarding the feel during use of the composition of the present invention, an addition of an oil-based gelling agent into the composition of the present invention may improve the thickness feeling and the richness feeling while unexpectedly reducing the stickiness feeling and improving the fresh feeling. The addition of an oil-based gelling agent may increase the viscosity of the oil phase, may delay an oil release after the application of the composition of the present invention to control the spreadability of oil, which may cause a fresh feeling in addition to a thickness feeling and a smoothness feeling.

Examples of an oil-based gelling agent include an inulin fatty acid ester (for example, stearoyl inulin), a dextrin fatty acid ester (for example, dextrin myristate), polyglycerol, dibutyl ethylhexanoyl glutamide, dibutyl lauroyl glutamide, sodium dilauramidoglutamide lysine, vinyl dimethicone/methicone silsesquioxane crosspolymer,and PEG/PPG-19/19 dimethicone.

A single kind of the oil-based gelling agent may be used, or any combination of two or more kinds of the oil-based gelling agent may be used.

Regarding the feel during use of the composition of the present invention, an addition of a silicone agent for improving feel into the composition of the present invention may alleviate the stickiness feeling, may increase the fresh feeling, and may unexpectedly increase the richness feeling and the thickness feeling.

Examples of a silicone agent for improving feel include an amino-modified silicone (for example, aminopropyl dimethicone), a silicone wax, polyether-modified silicone, highly polymerized dimethicone, a silicone wax (for example, stearyl dimethicone, bis-PEG-18 methyl ether dimethyl silane, C26-28 alkyl dimethicone, C26-28 alkyl methicone, bis-stearoxydimethylsilane, acrylates/stearyl acrylate/dimethicone methacrylate copolymer, acrylates/behenyl acrylate/dimethicone methacrylate copolymer a silicone emulsion, and a silicone powder. A single kind of the silicone agent for improving feel may be used, or any combination of two or more kinds of the silicone agent for improving feel may be used.

The amount of the Ingredient (G) is not particularly limited as long as the emulsification stability and/or the feel during use of the composition are good, but for example, the amount of Ingredient (G) in Ingredient (E) is 0.1 to 50% by weight, preferably 0.5 to 30% by weight, more preferably 1 to 20% by weight, still more preferably 2 to 13% by weight relative to Ingredient (E) 100% by weight.

Further, examples of the lower limit of the amount range of Ingredient (G) relative to Ingredient (E) 100% by weight include 0.1% by weight, 0.5% by weight, 1% by weight, 2% by weight, and examples of the upper limit include 50% by weight, 40% by weight, 30% by weight, 20% by weight, 13% by weight, and preferred examples of the range may be shown by a combination of the lower limit and the upper limit.

Further, when Ingredient (G) is an uncrystallized high melting point oil having a melting point of 30 °C or higher, the amount of Ingredient (G) in Ingredient (E) is, for example, 0.1 to 30% by weight, preferably 0.1 to 20% by weight, more preferably 0.5 to 15% by weight, still more preferably 3 to 10% by weight relative to Ingredient (E) 100% by weight.

Further, examples of the lower limit of the amount range of Ingredient (G) relative to Ingredient (E) 100% by weight include 0.1% by weight, 0.5% by weight, 1% by weight, 2% by weight, 3% by weight, and examples of the upper limit include 30% by weight, 20% by weight, 15% by weight, 10% by weight, 5% by weight, and preferred examples of the range may be shown by a combination of the lower limit and the upper limit.

Further, when Ingredient (G) is an oil-based gelling agent, the amount of Ingredient (G) in Ingredient (E) is, for example, 0.01 to 30% by weight, preferably 0.1 to 20% by weight, more preferably 0.5 to 10% by weight, still more preferably 2 to 7% by weight relative to Ingredient (E) 100% by weight.

Further, examples of the lower limit of the amount range of Ingredient (G) relative to Ingredient (E) 100% by weight include 0.01% by weight, 0.1% by weight, 0.5% by weight, 1% by weight, 2% by weight, and examples of the upper limit include 30% by weight, 20% by weight, 15% by weight, 10% by weight, 7% by weight, and preferred examples of the range may be shown by a combination of the lower limit and the upper limit.

Further, when Ingredient (G) is a silicone agent for improving feel, the amount of Ingredient (G) in Ingredient (E) is, for example, 0.01 to 50% by weight, preferably 0.1 to 30% by weight, more preferably 0.1 to 20% by weight, still more preferably 1 to 20% by weight, still even more preferably 3 to 15% by weight relative to Ingredient (E) 100% by weight.

Further, examples of the lower limit of the amount range of Ingredient (G) relative to Ingredient (E) 100% by weight include 0.01% by weight, 0.1% by weight, 0.5% by weight, 1% by weight, 2% by weight, 3% by weight, and examples of the upper limit include 50% by weight, 30% by weight, 20% by weight, 15% by weight, and preferred examples of the range may be shown by a combination of the lower limit and the upper limit.

The amount of Ingredient (G) which may be comprised in the composition of the present invention may be optionally selected from a range of, for example, usually 0.001 to 20% by weight based on the total weight of the composition. Preferably 0.01% by weight to 10% by weight, more preferably 0.1% by weight to 7% by weight, more preferably 0.5% by weight to 5% by weight, even more preferably 0.7% by weight to 3% by weight, still more preferably 0.3% by weight to 2% by weight are exemplified.

Further examples of the lower limit of the amount range of Ingredient (G) which may be comprised in the composition of the present invention include 0.001% by weight, 0.01% by weight, 0.1% by weight, 0.5% by weight, 0.7% by weight, 1% by weight, and examples of the upper limit include 20% by weight, 10% by weight, 7% by weight, 5% by weight, 3% by weight, 2% by weight, and preferred examples of the range may be shown by a combination of the lower limit and the upper limit.

The composition of the present invention may comprise Ingredient (H): a water-soluble humectant. The addition of a water-soluble humectant may improve the feel during use of the composition of the present invention.

Examples of a water-soluble humectant include a low-polarity polyhydric alcohol (for example, butylene glycol, bis-ethoxydiglycol cyclohexane 1,4-dicarboxylate, dipropylene glycol, PEG/PPG/polybutylene glycol-8/5/3 glycerin, POE methylglucoside), a saccharide (for example, glyceryl glucoside, diglycerin, trehalose, a sorbitol culture polysaccharide solution), and a film forming agent (for example, pullulan, PVP, gum arabic), and a single kind of the water-soluble humectant may be used, or any combination of two or more kinds of the water-soluble humectant may be used.

The amount of Ingredient (H) which may be comprised in the composition of the present invention may be optionally selected from, for example, a range of 0.001 to 50% by weight based on the total weight of the composition. Preferably 0.01% by weight to 30% by weight, more preferably 0.1% by weight to 20% by weight, more preferably 0.5% by weight to 10% by weight are exemplified.

Further, examples of the lower limit of the amount range of Ingredient (H) which may be comprised in the composition of the present invention include 0.001% by weight, 0.01% by weight, 0.1% by weight, 0.5% by weight, and 1% by weight, and examples of the upper limit include 1% by weight, 2% by weight, 3% by weight, 5% by weight, 7% by weight, 10% by weight, 20% by weight, 25% by weight, and 30% by weight, and preferred examples of the range may be shown by a combination of the lower limit and the upper limit.

The amount of water of Ingredient (F) comprised in the composition of the present invention is not particularly limited and may vary depending on the amounts of other ingredients, but may be optionally selected from, for example, a range of usually 20 to 99% by weight based on the total weight of the composition. Preferably 30% by weight to 99% by weight, more preferably 40% by weight to 99% by weight, more preferably 50% by weight to 99% by weight, even more preferably 60% by weight to 95% by weight, still more preferably 65% by weight to 90% by weight are exemplified.

Further, examples of the lower limit of the amount range of Ingredient (F) comprised in the composition of the present invention include 20% by weight, 30% by weight, 40% by weight, 50% by weight, 60% by weight, and 65% by weight, and examples of the upper limit include 99% by weight, 95% by weight, and 90, and preferred examples of the range may be shown by a combination of the lower limit and the upper limit.

The composition of the present invention may optionally comprise a chelating agent (for example, edetate, etidronic acid, sodium polyphosphate, sodium metaphosphate).

Each ingredient that may be compounded into the composition of the present invention may be in the form of a hydrate.

The composition of the present invention may be prepared in various forms by combining pharmaceutically or cosmetically acceptable base(s) or carrier(s) in addition to the above ingredients. For pharmaceutically or cosmetically acceptable base(s) and carrier(s), conventionally known one(s) can be used. The composition of the present invention may comprise, if required, a wide variety of known ingredients used for externally-applied compositions suitable for the skin or mucous membranes, such as cosmetics or externally-applied medical/quasi-medical drugs. Examples of such ingredients include solvents (for example, cyclomethicone, trimethylsiloxysilicate, C12-15 alkyl benzoate, diethylamino hydroxybenzoyl hexyl benzoate), detergent bases (for example, PEG-7 glyceryl cocoate), surfactants, colorants (dyes and pigments), flavors, preservatives, bactericides ((antibacterials), for example, phenoxyethanol, paraben), thickeners (for example, carboxyvinylpolymer, pentaerythrityl tetraethylhexanoate, pentaerythrityl tetrapentahexanoate), antioxidants, sequestering agents, cooling agents, deodorizers, humectants (for example, glycerin, methyl gluceth-10, pentylene glycol), UV absorbers (for example, 2-ethylhexyl p-methoxycinnamate, octocrylene), UV dispersants, vitamins, plant extracts, skin astringents (for example, ethanol), anti-inflammatory agents (antiphlogistic agents), whitening agents, cell activators, vasodilators, blood circulation accelerators and skin function accelerators.

The composition of the present invention may be used as a composition for external use to be applied or sprayed on the skin. Specifically, the composition of the present invention may be used as an external preparation (skin preparation) for cosmetics, external medicines or external quasi-medicines.

The form of the composition of the present invention is not particularly limited as long as it can be applied to the skin, and examples thereof include a paste, a mousse, a gel, a liquid, a milky liquid, a suspension liquid, a cream, an ointment, a solid, a sheet, an aerosol, a spray, and a liniment. Especially when using as a cosmetic, lotion; emulsions such as emollient emulsion, milky lotion, nourishing emulsion, and cleansing emulsion; cream such as emollient cream, massage cream, cleansing cream, and makeup cream; or a lip balm.

### Example

The present invention is explained in further detail with reference to Formulation Examples and Test Examples.

### Test Example 1

Each of the ingredients were combined together and mixed according to the compositions shown in Table 1 to give skin emulsion compositions of Examples 1 and 2 and Comparative examples 1 to 5. Specifically, while ingredients 1 to 5 and 7 shown in the table were mixed, the pH of the mixture was adjusted by an addition of ingredient 6, and the mixture was uniformly mixed to give a gel. The resulting gel was uniformly mixed with ingredient 8 to give a emulsion composition. Viscosity and pH of the gel (before emulsification) and the emulsion composition (after emulsification) were measured.

The pH was measured at a room temperature of 25 °C using a pH meter F-52 (manufactured by HORIBA, Ltd.).

The viscosity was measured by B-type viscometer.

Specifically, the viscosity was measured at a room temperature of 25 °C using BM type viscometer (manufactured by Tokyo Keiki) (rotor No. 2, 3 and 4, 12 rpm, 60 seconds). The unit is mPa·s. The emulsified state was visually evaluated (X: Non-emulsifiable, △: Insufficient emulsification, O: Emulsifiable).

The results are shown in Table 1.

FIG. 1 is a graph showing the viscosity of the gel before emulsification.

**[Table 1]**

| Ingredient (% by weight) | | Comp. Ex. 1 | Comp. Ex. 2 | Comp. Ex. 3 | Comp. Ex. 4 | Ex. 1 | Comp. Ex. 5 | Ex. 2 |
|---|---|---|---|---|---|---|---|---|
| 1 | Purified water | Balan ce | Balan ce | Balan ce | Balan ce | Balan ce | Balan ce | Balan ce |
| 2 | Acrylates/C10-30 alkyl acrylate crosspolymer | 0.4 | - | 0.4 | - | 0.4 | - | 0.4 |
| 3 | Carboxyvinylpoly mer | - | 0.2 | 0.2 | - | - | - | - |
| 4 | PEG-240/HDI copolymer bis-decyltetradeceth -20 ether | - | - | - | 1.5 | 1.5 | 0.6 | 0.6 |
| 5 | Adenosine phosphate | 2 | - | 2 | - | 2 | - | 2 |
| 6 | Potassium hydroxide | q.s | q.s | q.s | q.s | q.s | q. s | q.s |
| 7 | BG | - | 2.5 | 2.5 | 2.5 | 2.5 | 2.5 | 2.5 |
| 8 | Liquid paraffin | 5 | - | 5 | - | 5 | - | 5 |
| Total | | | 100 | 100 | 100 | 100 | 100 | 100 |
| Viscosity Before emulsification | | 1750 | 30750 | 5750 | 28300 | 88000 | 35 | 17500 |
| Viscosity After emulsification | | 1000 | Non-emuls ifiab le | 4550 | Non-emuls ifiab le | 11040 0 | Non-emuls ifiab le | 15750 |
| Emulsified state | | X | X | △ | X | O | X | O |
| pH | | 6.5 to 7.0 | 6.5 to 7.0 | 6.5 to 7.0 | 6.5 to 7.0 | 6.5 to 7.0 | 6.5 to 7.0 | 6.5 to 7.0 |

| | | | | | | | | |
|---|---|---|---|---|---|---|---|---|
| Comp. Ex.: Comparative example Ex.: Example q.s: quantum sufficit | | | | | | | | |

As shown in Table 1, Examples 1 and 2 exhibited a high viscosity of equal to or higher than 10,000 mPa·s, and the emulsified state was good. The viscosity results of the gel before emulsification indicated that the PEG-240/HDI copolymer bis-decyltetradeceth-20 ether significantly improved the viscosity of the composition comprising adenosine phosphate and acrylates/C10-30 alkyl acrylate crosspolymer.

### Test Example 2

Each of the ingredients were combined together and mixed according to the compositions shown in Table 2 to give skin emulsion compositions of Examples 3 and 4 and Comparative example 6. Specifically, while ingredients 1 to 4 and 8 to 12 shown in the table were mixed, the pH of the mixture was adjusted by an addition of one of ingredients 5 to 7, and the mixture was uniformly mixed to give a gel. The resulting gel was uniformly mixed with ingredients 13 to 15 to give a emulsion composition.

The pH and the viscosity were determined in the same manner as in Test Example 1. In addition, the resulting emulsion compositions were placed in a constant temperature chamber at 60 °C and allowed to stand for 1 weeks, and then the color and odor were evaluated by a sensory evaluation. (△: No improvement, O: Slight improvement, ⊚: Marked improvement)

**[Table 2]**

| Ingredient (% by weight) | | Comp. Ex. 6 | Ex. 3 | Ex. 4 |
|---|---|---|---|---|
| 1 | Purified water | Balance | Balance | Balance |
| 2 | Acrylates/C10-30 acrylate crosspolymer | 0.8 | 0.8 | 0.8 |
| 3 | Carboxyvinylpolymer | 0.1 | 0.1 | 0.1 |
| 4 | Adenosine phosphate | 2 | 2 | 2 |
| 5 | Potassium hydroxide | q.s | - | - |
| 6 | Tromethamine | - | q.s | - |
| 7 | Arginine | - | - | q.s |
| 8 | Glycerin | 5 | 5 | 5 |
| 9 | Methyl gluceth-10 | 3 | 3 | 3 |
| 10 | Pentylene glycol | 2 | 2 | 2 |
| 11 | Phenoxyethanol | 0.2 | 0.2 | 0.2 |
| 12 | Edetate | 0.05 | 0.05 | 0.05 |
| 13 | Cyclopentasiloxane | 3 | 3 | 3 |
| 14 | Pentaerythrityl tetrapentahexanoate | 5 | 5 | 5 |
| 15 | Squalane | 6.5 | 6.5 | 6.5 |
| Total | | 100 | 100 | 100 |
| Viscosity | | 33700 | 38500 | 40250 |
| pH | | 6.5 | 6.5 | 6.5 |
| Odor change | | - | ⊚ | ○ |
| Discoloration | | - | ⊚ | △ |

| | | | | |
|---|---|---|---|---|
| Comp. Ex.: Comparative example Ex.: Example q.s: quantum sufficit | | | | |

As shown in Table 2, Examples 3 and 4 comprising tromethamine or arginine, both of which are organic alkali compounds, showed significantly higher viscosity values than that of Comparative Example 6 comprising potassium hydroxide, which is an inorganic alkali compound. In terms of discoloration, Examples 3 and 4 showed results equivalent to or better than that of Comparative example 6. In terms of odor change, Examples 3 and 4 showed better results than that of Comparative example 6.

### Test Example 3-1

Each of the ingredients were combined together and mixed according to the compositions shown in Table 3-1 to give skin emulsion compositions of Examples 5 to 9 and Comparative examples 7 to 8. Specifically, while ingredients 1 to 4 and 6 to 8 shown in the table were mixed, the pH of the mixture was adjusted by an addition of the ingredient 5, and the mixture was uniformly mixed to give a gel. The resulting gel was uniformly mixed with ingredients 9 to 13 and emulsified.

A sensory evaluation of Examples 5 to 9 and Comparative examples 7 to 8 was performed by 4 panelists.

As for the feel during use,
the more thickness feeling is, the better result is;
the more richness feeling is, the better result is;
the less stickiness feeling is, the better result is;
the more fresh feeling is, the better result is.

### <Sensory Evaluation Score>

The same amount of the test composition (0.1 g) was spread on the inner side of the forearm with a constant force, and the thickness feeling, the richness feeling, the less stickiness feeling, the fresh feeling were scored as below, using point 3 of Comparative example 7 as a reference point. The average points based on 4 panelists were calculated.

| | |
|---|---|
| 5 points: | Markedly improved |
| 4 points: | Improved |
| 3 points: | No difference |
| 2 points: | Worsened |
| 1 point : | Markedly worsened |

The total sum of each of the average points of the thickness feeling, the richness feeling, the less stickiness feeling, and the fresh feeling was used as an overall score for each sample. A comprehensive evaluation of the feeling during use was rated based on this overall score as follows.

### <Comprehensive evaluation>

"⊚" represents that overall score was 17 or more, which shows that the feel during use was improved.
"O" represents that overall score was 13 or more but less than 17, which shows that the feel during use was slightly improved.
"△" represents that overall score was 10 or more but less than 13, which shows that no improvement in the feel during use was perceived.
"X" represents that overall score was less than 10, which shows that the feel during use was worsened.

**[Table 3-1]**

| Ingredient (% by weight) | | Comp. Ex. 7 | Comp. Ex. 8 | Ex. 5 | Ex. 6 | Ex. 7 | Ex. 8 | Ex. 9 |
|---|---|---|---|---|---|---|---|---|
| 1 | Purified water | Balan ce | Balan ce | Balan ce | Balan ce | Balan ce | Balan ce | Balan ce |
| 2 | Acrylates/C10-30 alkyl acrylate crosspolymer | 0.8 | 0.8 | 0.7 | 0.8 | 0.8 | 0.8 | 0.8 |
| 3 | Carboxyvinylpoly mer | - | - | 0.1 | - | - | - | - |
| 4 | Adenosine phosphate | 2 | 2 | 2 | 2 | 2 | 2 | 2 |
| 5 | Tromethamine | q.s | q.s | q.s | q.s | q.s | q.s | q.s |
| 6 | Glycerin | 5 | 5 | 5 | 5 | 5 | 5 | 5 |
| 7 | Diglycerin | 2 | 2 | 2 | 2 | 2 | 2 | 2 |
| 8 | Pentylene glycol | 3 | 3 | 3 | 3 | 3 | 3 | 3 |
| 9 | Liquid paraffin | 10 | 8.5 | 10 | 9.5 | 9 | 9 | 8.5 |
| 10 | Pentaerythrityl tetraethylhexano ate | 5 | 5 | 5 | 5 | 5 | 5 | 5 |
| 11 | Cetyl alcohol | - | 1.5 | - | - | - | - | - |
| 12 | Caprylic/capric/ myristic/stearic triglyceride | - | - | - | 0.5 | 1 | - | 0.5 |
| 13 | Phytosteryl/behe nyl/octyldodecyl lauroyl glutamate | - | - | - | - | - | 1 | 1 |
| pH | | 6.3 | 6.3 | 6.3 | 6.3 | 6.3 | 6.3 | 6.3 |
| Viscosity | | 29200 | N/A | 29000 | 35750 | 32700 | 31900 | 33500 |
| Thickness feeling | | 3 | N/A | 3 | 4.5 | 4.8 | 4.5 | 5 |
| Richness feeling | | 3 | N/A | 3 | 4.5 | 5 | 5 | 4.8 |
| Less stickiness feeling | | 3 | N/A | 3 | 3.5 | 4.3 | 3.8 | 4.5 |
| Fresh feeling | | 3 | N/A | 4.5 | 4.5 | 4.5 | 3.8 | 4.8 |
| Overall score | | 12 | | 13.5 | 17 | 18.6 | 17.1 | 19.1 |
| Comprehensive evaluation | | - | N/A | △ | ⊚ | ⊚ | ⊚ | ⊚ |

| | | | | | | | | |
|---|---|---|---|---|---|---|---|---|
| Comp. Ex.: Comparative example Ex.: Example q.s: quantum sufficit N/A: Not available since a uniform composition of Comparative example 8 was unpreparable. | | | | | | | | |

As shown in Table 3-1, Examples 6 to 9 comprises caprylic/capric/myristic/stearic triglyceride or phytosteryl/behenyl/octyldodecyl lauroyl glutamate, all of which are uncrystallized high melting point oils. As shown in Table 3-1, Examples 6 to 9 comprising an uncrystallized high melting point oil each showed an improvement in the feel during use compared with Comparative example 7, which does not comprise an uncrystallized high melting point oil. Comparative Example 8 in which cetyl alcohol, a crystallized high melting point oil, was comprised, did not form a uniform composition.

### Test Example 3-2

Each of the ingredients were combined together and mixed according to the compositions shown in Table 3-2 to give the skin emulsion compositions of Comparative example 9, Examples 10 to 13. The pH and viscosity of the resulting emulsion composition for skin were determined in the same manner as in Test Example 1, and the feeling during use was evaluated in the same manner as in Test Example 3-1.

**[Table 3-2]**

| Ingredient (% by weight) | | Comp. Ex. 9 | Ref. Ex. 10 | Ref. Ex. 11 | Ref. Ex. 12 | Ref. Ex. 13 |
|---|---|---|---|---|---|---|
| 1 | Purified water | Balance | Balance | Balance | Balance | Balance |
| 2 | Acrylates/C10-30 alkyl acrylate crosspolymer | 0.8 | 0.8 | 0.8 | 0.8 | 0.8 |
| 3 | Adenosine phosphate | 2 | 2 | 2 | 2 | 2 |
| 4 | Potassium hydroxide | q.s | q.s | q.s | q.s | q.s |
| 5 | Glycerin | 5 | 5 | 5 | 5 | 5 |
| 6 | Diglycerin | 2 | 2 | 2 | 2 | 2 |
| 7 | Pentylene glycol | 3 | 3 | 3 | 3 | 3 |
| 8 | Pentaerythrityl tetraethylhexanoate | 10 | 9.7 | 9 | 9.7 | 9 |
| 9 | Liquid paraffin | 5 | 5 | 5 | 5 | 5 |
| 10 | Stearoyl inulin | - | 0.3 | 1 | - | - |
| 11 | Dextrin myristate | - | - | - | 0.3 | 1 |
| pH | | 6.5 to 7.0 | 6.5 to 7.0 | 6.5 to 7.0 | 6.5 to 7.0 | 6.5 to 7.0 |
| Viscosity | | 29200 | 39750 | 41400 | 39800 | 38000 |
| Thickness feeling | | 3 | 4 | 4.8 | 3.8 | 4.5 |
| Richness feeling | | 3 | 4 | 4.5 | 4 | 4.8 |
| Less stickiness feeling | | 3 | 4.3 | 4.8 | 4 | 4.3 |
| Fresh feeling | | 3 | 4.5 | 5 | 4 | 5 |
| Overall score | | 12 | 16.8 | 19.1 | 15.8 | 18.6 |
| Comprehensive evaluation | | X | ○ | ⊚ | ○ | ⊚ |

| | | | | | | |
|---|---|---|---|---|---|---|
| Comp. Ex.: Comparative example Ref. Ex.: Reference Example q.s: quantum sufficit | | | | | | |

As shown in Table 3-2, Reference Examples 10 to 13 comprise stearoyl inulin or dextrin myristate, both of which an oil-based gelling agent. Reference Examples 10 to 13 comprising an oil-based gelling agent each showed an improvement in the feel during use compared with Comparative Example 9, which does not comprise an oil-based gelling agent.

### Test Example 3-3

Each of the ingredients were combined together and mixed according to the compositions shown in Table 3-3 to give skin emulsion compositions of Comparative example 10, Examples 14 to 16.

The pH and viscosity of the resulting emulsion compositions for skin were determined in the same manner as in Test Example 1, and the feeling during use was evaluated in the same manner as in Test Example 3-1.

**[Table 3-3]**

| Ingredient (% by weight) | | Comp. Ex. 10 | Ex. 14 | Ex. 15 | Ex. 16 |
|---|---|---|---|---|---|
| 1 | Purified water | Balance | Balance | Balance | Balance |
| 2 | Acrylates/C10-30 alkyl acrylate crosspolymer | 0.6 | 0.6 | 0.6 | 0.6 |
| 3 | Carboxyvinylpolymer | 0.2 | 0.2 | 0.2 | 0.2 |
| 4 | Adenosine phosphate | 2 | 2 | 2 | 2 |
| 5 | Tromethamine | q.s | q.s | q.s | q.s |
| 6 | Liquid paraffin | - | - | - | - |
| 7 | Cyclopentasiloxane | 15 | 14.5 | 14.5 | 13 |
| 8 | Highly polymerized dimethicone | - | 0.5 | - | - |
| 9 | Stearyl dimethicone | - | - | 0.5 | 2 |
| pH | | 6.4 | 6.4 | 6.4 | 6.4 |
| Viscosity | | 22250 | 21650 | 22250 | 22400 |
| Thickness feeling | | 3 | 5 | 4.3 | 5 |
| Richness feeling | | 3 | 5 | 4.8 | 5 |
| Less stickiness feeling | | 3 | 4 | 4.3 | 4.8 |
| Fresh feeling | | 3 | 3.8 | 3.8 | 4 |
| Overall score | | 3 | 17.8 | 17.2 | 18.8 |
| Comprehensive evaluation | | X | ⊚ | ⊚ | ⊚ |

| | | | | | |
|---|---|---|---|---|---|
| Comp. Ex.: Comparative example Ex.: Example q.s: quantum sufficit | | | | | |

As shown in Table 3-3, Examples 14 to 16 comprise highly polymerized dimethicone or stearyl dimethicone, both of which are silicone agents for improving feel. Examples 14 to 16 comprising a silicone agent for improving feel each showed an improvement in the feel during use compared with Comparative example 10, which does not comprise a silicone agent for improving feel.

### Formulation Examples of Oil-in-Water Emulsified Cosmetics

All amounts are expressed in % by weight based on the total weight of the product.

### <1. Gel cream>

| | | |
|---|---|---|
| 1 | Adenosine phosphate | 3.0 |
| 2 | Acrylates/C10-30 alkyl acrylate crosspolymer | 1 |
| 3 | Pentylene glycol | 3 |
| 4 | Diglycerin | 1 |
| 5 | Glycerin | 4 |
| 6 | Pullulan | 0.5 |
| 7 | 1,3-butylene glycol | 2 |
| 8 | POE methylglucoside | 3 |
| 9 | Liquid paraffin | 6 |
| 10 | Phytosteryl/octyldodecyl lauroyl glutamate | 0.2 |
| 11 | Pentaerythrityl tetraethylhexanoate | 12 |
| 12 | Dextrin myristate | 0.3 |
| 13 | Tromethamine | q.s |
| 14 | Phenoxyethanol | 0.3 |
| 15 | Purified water | Balance |
| | TOTAL | 100 |

### <2. Gel cream> (Reference Example)

| | | |
|---|---|---|
| 1 | Adenosine phosphate | 2.0 |
| 2 | Acrylates/C10-30 alkyl acrylate crosspolymer | 0.7 |
| 3 | Xanthan gum | 0.1 |
| 4 | Carboxyvinylpolymer | 0.1 |
| 5 | A sorbitol culture polysaccharide solution | 1 |
| 6 | POE methylglucoside | 2 |
| 7 | BG | 8 |
| 8 | Glycerin | 4 |
| 9 | Cyclopentasiloxane | 5 |
| 10 | Highly polymerized dimethicone | 0.5 |
| 11 | Potassium hydroxide | q.s |
| 12 | Paraben | 0.25 |
| 13 | Purified water | Balance |
| | TOTAL | 100 |

### <3. Sunscreen cream>

| | | |
|---|---|---|
| 1 | Adenosine phosphate | 1 |
| 2 | Cyclomethicone | 8 |
| 3 | Trimethylsiloxysilicate | 0.4 |
| 4 | C12-15 alkyl benzoate | 2 |
| 5 | 2-ethylhexyl p-methoxycinnamate | 7 |
| 6 | Octocrylene | 2 |
| 7 | Diethylamino hydroxybenzoyl hexyl benzoate | 3 |
| 8 | Acrylates/C10-30 alkyl acrylate crosspolymer | 0.6 |
| 9 | PEG-240/HDI copolymer bis-decyltetradeceth-20 ether | 1.5 |
| 10 | Concentrated glycerin | 4 |
| 11 | 1,3-butylene glycol | 3 |
| 12 | Ethanol | 7 |
| 13 | Potassium hydroxide | q.s |
| 14 | Phenoxyethanol | 0.3 |
| 15 | Purified water | Balance |
| | TOTAL | 100 |

### <4. Cleansing cream> (Reference Example)

| | | |
|---|---|---|
| 1 | Adenosine phosphate | 0.5 |
| 2 | Cyclomethicone | 10 |
| 3 | PEG-7 glyceryl cocoate | 7 |
| 4 | Stearoyl inulin | 0.4 |
| 5 | Acrylates/C10-30 alkyl acrylate crosspolymer | 0.6 |
| 6 | Acrylates/steareth-20 methacrylate crosspolymer | 0.4 |
| 7 | 1,3-butylene glycol | 15 |
| 8 | Potassium hydroxide | q.s |
| 9 | Paraben | 0.3 |
| 10 | Purified water | Balance |
| | TOTAL | 100 |

## Claims

1. An O/W emulsion composition,
comprising Ingredient (A), Ingredient (B), Ingredient (E), and Ingredient (F); and
comprising Ingredient (C) and/or Ingredient (D);
wherein
| | |
|---|---|
| Ingredient (A): | an adenosine phosphate and/or a salt thereof; |
| Ingredient (B): | an acrylic acid-alkyl methacrylate copolymer; |
| Ingredient (C): | a water-soluble polymer which is a urethane associative thickener; |
| Ingredient (D): | a pH adjuster which is an organic alkali compound selected from tromethamine, aminomethyl propanol, arginine, and a mixture thereof; |
| Ingredient (E): | an oil content; |
| Ingredient (F): | water, and |
wherein the composition has a viscosity , determined according to the method in the description, of equal to or higher than 10,000 mPa·s.

2. The composition according to Claim 1, wherein Ingredient (B) is an acrylic acid-alkyl methacrylate copolymer having a molecular weight of 100,000 to 5,000,000.

3. The composition according to Claim 1 or 2, wherein Ingredient (D) is a pH adjuster which is an organic alkali compound selected from tromethamine, arginine, and a mixture thereof.

4. The composition according to any one of Claims 1 to 3, wherein Ingredient (C) is a water-soluble polymer which is a urethane associative thickener selected from the group consisting of PEG-240/HDI copolymer bis-decyltetradeceth-20 ether, PEG-150/stearyl alcohol/SMDI copolymer, PEG-150/decyl alcohol/SMDI copolymer, polyurethane-59 and a mixture thereof.

5. The composition according to any one of Claims 1 to 4, wherein the composition has a pH of 5.5 to 7.5.

6. The composition according to any one of Claims 1 to 5 comprising
| | | |
|---|---|---|
| Ingredient | (A): | an adenosine phosphate and/or a salt |
| Ingredient | (B) : | acrylates/C10-30 alkyl acrylate crosspolymer; |
| Ingredient | (D) : | tromethamine and/or arginine; |
| Ingredient | (E) : | an oil content; and |
| Ingredient | (F) : | water, |
wherein the composition has a viscosity, determined according to the method in the description, of 20,000 tc 40,000 mPa·s, and a pH of 6 to 7.

7. The composition according to any one of Claims 1 to 6, comprising
| | | |
|---|---|---|
| Ingredient | (A): | adenosine phosphate and/or a salt thereof; |
| Ingredient | (B): | acrylates/C10-30 alkyl acrylate crosspolymer; |
| Ingredient | (C): | PEG-240/HDI copolymer bis-decyltetradeceth-20 ether; |
| Ingredient | (E): | an oil content; and |
| Ingredient | (F): | water, |
wherein the composition has a viscosity, determined according to the method in the description, of 20,000 tc 40,000 mPa·s.

8. The composition according to any one of Claims 1 to 7, wherein the composition comprises
0.001 to 10% by weight of Ingredient (A),
0.05 to 5% by weight of Ingredient (B), and
1 to 60% by weight of Ingredient (E).

9. The composition according to any one of Claims 1 to 8, wherein the composition comprises 0.01 to 20% by weight of Ingredient (C).

10. The composition according to any one of Claims 1 to 9 comprising
| | | | |
|---|---|---|---|
| Ingredient | (A): | an adenosine phosphate and/or a salt thereof | 1.5 to 3.5% by weight; |
| Ingredient | (B): | acrylates/C10-30 alkyl acrylate crosspolymer | 0.4 to 0.8% by weight; |
| Ingredient | (D): | tromethamine and/or arginine; | |
| Ingredient | (E): | an oil content | 5 to 20% by weight; and |
| Ingredient | (F): | water, | |
wherein the composition has a viscosity, determined according to the method in the description, of 20,000 tc 40,000 mPa·s, and a pH of 6 to 7.

11. The composition according to any one of Claims 1 to 10 comprising
| | | | |
|---|---|---|---|
| Ingredient | (A): | an adenosine phosphate and/or a salt thereof | 1.5 to 3.5% by weight; |
| Ingredient | (B): | acrylates/C10-30 alkyl acrylate crosspolymer | 0.4 to 0.8% by weight; |
| Ingredient | (C): | PEG-240/HDI copolymer bis-decyltetradeceth-20 ether | 0.3 to 0.8% by weight; |
| Ingredient | (E): | an oil content and | 5 to 20% by weight; |
| Ingredient | (F): | water, | |
wherein the composition has a viscosity, determined according to the method in the description, of 20,000 tc 40,000 mPa·s.

12. The composition according to any one of Claims 1 to 11, wherein
the composition does not comprise a pH adjuster which is an inorganic alkali compound, or ,
the composition comprises a pH adjuster which is an inorganic alkali compound, and the pH adjuster which is an inorganic alkali compound/Ingredient (D) (weight ratio) is 0.1 or less.

13. The composition according to any one of 1 to 12,
wherein Ingredient (E) comprises Ingredient (G) selected from:
an uncrystallized high melting point oil having a melting point of 30 °C or higher selected from an uncrystallized triglyceride having a melting point of 30 °C or higher; an uncrystallized sterol fatty acid ester having a melting point of 30 °C or higher; petrolatum; and a mixture thereof;
an oil-based gelling agent selected from an inulin fatty acid ester, a dextrin fatty acid ester, polyglycerol, dibutyl ethylhexanoyl glutamide, dibutyl lauroyl glutamide, sodium dilauramidoglutamide lysine, vinyl dimethicone/methicone silsesquioxane crosspolymer, PEG/PPG-19/19 dimethicone, and a mixture thereof;
a silicone agent for improving feel selected from an amino-modified silicone, a silicone wax, polyether-modified silicone, highly polymerized dimethicone, a silicone wax, a silicone emulsion, a silicone powder, and a mixture thereof; and
a mixture thereof, and
wherein the amount of Ingredient (G) is 0.1 to 50% by weight based on Ingredient (E).

14. The composition according to any one of Claims 1 to 13 wherein the composition comprises 0.001 to 20% by weight of Ingredient (G).

15. The composition according to any one of Claims 1 to 14, wherein the composition further comprises
Ingredient (H): a water-soluble humectant.

## Patentansprüche

1. O/W-Emulsionszusammensetzung,
umfassend Inhaltsstoff (A), Inhaltsstoff (B), Inhaltsstoff (E) und Inhaltsstoff (F) und
umfassend Inhaltsstoff (C) und/oder Inhaltsstoff (D),
wobei
| | | |
|---|---|---|
| Inhaltsstoff | (A): | ein Adenosinphosphat und/oder ein Salz davon, |
| Inhaltsstoff | (B): | ein Acrylsäure-Alkylmethacrylat-Copolymer, |
| Inhaltsstoff | (C): | ein wasserlösliches Polymer, das ein Urethan-assoziativer Verdicker ist, |
| Inhaltsstoff | (D): | ein pH-Einstellungsmittel, das eine organische Alkaliverbindung, ausgewählt aus Tromethamin, Aminomethylpropanol, Arginin und einer Mischung davon, |
| ist, | | |
| Inhaltsstoff | (E): | einen Ölgehalt, |
| Inhaltsstoff | (F): | Wasser und |
wobei die Zusammensetzung eine Viskosität, bestimmt gemäß dem Verfahren in der Beschreibung, von gleich oder höher als 10.000 mPa·s aufweist.

2. Zusammensetzung gemäß Anspruch 1, wobei Inhaltsstoff (B) ein Acrylsäure-Alkylmethacrylat-Copolymer mit einem Molekulargewicht von 100.000 bis 5.000.000 ist.

3. Zusammensetzung gemäß Anspruch 1 oder 2, wobei Inhaltsstoff (B) ein pH-Einstellungsmittel ist, das eine organische Alkaliverbindung, ausgewählt aus Tromethamin, Arginin und einer Mischung davon, ist.

4. Zusammensetzung gemäß einem der Ansprüche 1 bis 3, wobei Inhaltsstoff (C) ein wasserlösliches Polymer ist, das ein Urethan-assoziatives Verdickungsmittel, ausgewählt aus der Gruppe, bestehend aus PEG-240/HDI-Copolymer-bisdecyltetradeceth-20-ether, PEG-150/Stearylalkohol/SMDI-Copolymer, PEG-150/Decylalkohol/SMDI-Copolymer, Polyurethan-59 und einer Mischung davon, ist.

5. Zusammensetzung gemäß einem der Ansprüche 1 bis 4, wobei die Zusammensetzung einen pH-Wert von 5,5 bis 7,5 aufweist.

6. Zusammensetzung gemäß einem der Ansprüche 1 bis 5, umfassend
| | | |
|---|---|---|
| Inhaltsstoff | (A): | ein Adenosinphosphat und/oder ein Salz davon, |
| Inhaltsstoff | (B): | Acrylate/C10-30-Alkylacrylat-Crosspolymer, |
| Inhaltsstoff | (D): | Tromethamin und/oder Arginin, |
| Inhaltsstoff | (E): | einen Ölgehalt und |
| Inhaltsstoff | (F): | Wasser, |
wobei die Zusammensetzung eine Viskosität, bestimmt gemäß dem Verfahren in der Beschreibung, von 20.000 bis 40.000 mPa·s und einen pH-Wert von 6 bis 7 aufweist.

7. Zusammensetzung gemäß einem der Ansprüche 1 bis 6, umfassend
| | | |
|---|---|---|
| Inhaltsstoff | (A): | ein Adenosinphosphat und/oder ein Salz davon, |
| Inhaltsstoff | (B): | ein Acrylate/C10-30-Alkylacrylat-Crosspolymer, |
| Inhaltsstoff | (C): | PEG-240/HDI-Copolymer-bisdecyltetradeceth-20-ether, |
| Inhaltsstoff | (E): | einen Ölgehalt und |
| Inhaltsstoff | (F): | Wasser |
wobei die Zusammensetzung eine Viskosität, bestimmt gemäß dem Verfahren in der Beschreibung, von 20.000 bis 40.000 mPa·s aufweist.

8. Zusammensetzung gemäß einem der Ansprüche 1 bis 7, wobei die Zusammensetzung
0,001 bis 10 Gew.-% Inhaltsstoff (A),
0,05 bis 5 Gew.-% Inhaltsstoff (B) und
1 bis 60 Gew.-% Inhaltsstoff (E) umfasst.

9. Zusammensetzung gemäß einem der Ansprüche 1 bis 8, wobei die Zusammensetzung 0,01 bis 20 Gew.-% Inhaltsstoff (C) umfasst.

10. Zusammensetzung gemäß einem der Ansprüche 1 bis 9, umfassend
| | | | |
|---|---|---|---|
| Inhaltsstoff | (A): | ein Adenosinphosphat und/oder ein Salz davon | 1,5 bis 3,5 Gew.-%, |
| Inhaltsstoff | (B): | Acrylate/C10-30-Alkylacrylat-Crosspolymer | 0,4 bis 0,8 Gew.-%, |
| Inhaltsstoff | (D): | Tromethamin und/oder Arginin, | |
| Inhaltsstoff | (E): | einen Ölgehalt | 5 bis 20 Gew.-% und |
| Inhaltsstoff | (F): | Wasser, | |
wobei die Zusammensetzung eine Viskosität, bestimmt gemäß dem Verfahren in der Beschreibung, von 20.000 bis 40.000 mPa·s und einem pH-Wert von 6 bis 7 aufweist.

11. Zusammensetzung gemäß einem der Ansprüche 1 bis 10, umfassend
| | | | |
|---|---|---|---|
| Inhaltsstoff | (A): | ein Adenosinphosphat und/oder ein Salz davon | 1,5 bis 3,5 Gew.-%, |
| Inhaltsstoff | (B): | Acrylate/C10-30-Alkylacrylat-Crosspolymer | 0,4 bis 0,8 Gew.-%, |
| Inhaltsstoff | (D): | PEG-240/HDI-Copolymer- bisdecyltetradeceth-20-ether | 0,3 bis 0,8 Gew.-%, |
| Inhaltsstoff | (E): | einen Ölgehalt | 5 bis 20 Gew.-% und |
| Inhaltsstoff | (F): | Wasser, | |
wobei die Zusammensetzung eine Viskosität, bestimmt gemäß dem Verfahren in der Beschreibung, von 20.000 bis 40.000 mPa·s aufweist.

12. Zusammensetzung gemäß einem der Ansprüche 1 bis 11, wobei
die Zusammensetzung kein pH-Einstellungsmittel umfasst, das eine anorganische Alkaliverbindung ist, oder
die Zusammensetzung ein pH-Einstellungsmittel umfasst, das eine anorganische Alkaliverbindung ist, und das pH-Einstellungsmittel, das eine anorganische Alkaliverbindung ist/Inhaltsstoff (D) (Gewichtsverhältnis) 0,1 oder weniger beträgt.

13. Zusammensetzung gemäß einem von 1 bis 12,
wobei Inhaltsstoff (E) Inhaltsstoff (G), ausgewählt aus:
einem unkristallisierten Öl mit hohem Schmelzpunkt, das einen Schmelzpunkt von 30°C oder höher aufweist, ausgewählt aus einem unkristallisierten Triglycerid mit einem Schmelzpunkt von 30°C oder höher, einem unkristallisierten Sterolfettsäureester mit einem Schmelzpunkt von 30° oder höher, Vaseline und eine Mischung davon,
einem Geliermittel auf Ölbasis, ausgewählt aus einem Inulinfettsäureester, einem Dextrinfettsäureester, Polyglycerin, Dibutylethylhexanoylglutamid, Dibutyllauroylglutamid, Natriumdilauramidoglutamidlysin, Vinyldimethicon/Methiconsilsesquioxan-Crosspolymer, PET/PPG-19/19-Dimethicon und einer Mischung davon,
einem Silikonmittel zur Verbesserung der Haptik, ausgewählt aus einem aminomodifizierten Silikon, einem Silikonwachs, polyethermodifiziertem Silikon, hochpolymerisiertem Dimethicon, einem Silikonwachs, einer Silikonemulsion, einem Silikonpulver und einer Mischung davon, und
einer Mischung davon, umfasst und
wobei die Menge an Inhaltsstoff (G) 0,1 bis 50 Gew.-% bezogen auf Inhaltsstoff (E), beträgt.

14. Zusammensetzung gemäß einem der Ansprüche 1 bis 13, wobei die Zusammensetzung 0,001 bis 20 Gew.-% Inhaltsstoff (G) umfasst.

15. Zusammensetzung gemäß einem der Ansprüche 1 bis 14, wobei die Zusammensetzung ferner umfasst
Inhaltsstoff (H): ein wasserlösliches Feuchthaltemittel.

## Revendications

1. Composition d'émulsion huile/eau,
comprenant un Ingrédient (A), un Ingrédient (B), un Ingrédient (E) et un Ingrédient (F) ; et
comprenant un Ingrédient (C) et/ou un Ingrédient (D) ;
dans laquelle
Ingrédient (A) : un phosphate d'adénosine et/ou un sel de celui-ci ;
Ingrédient (B) : un copolymère d'acide acrylique et de méthacrylate d'alkyle ;
Ingrédient (C) : un polymère soluble dans l'eau qui est un épaississant associatif de type uréthane ;
Ingrédient (D) : un correcteur de pH qui est un alcali organique sélectionné parmi la trométhamine, l'aminométhylpropanol, l'arginine, et un mélange de ceux-ci ;
Ingrédient (E) : un composant huileux ;
Ingrédient (F) : de l'eau, et
dans laquelle la composition présente une viscosité, déterminée selon la méthode décrite dans la description, supérieure ou égale à 10 000 mPa·s.

2. Composition selon la revendication 1, dans laquelle l'Ingrédient (B) est un copolymère d'acide acrylique et de méthacrylate d'alkyle présentant un poids moléculaire de 100 000 à 5 000 000.

3. Composition selon la revendication 1 ou revendication 2, dans laquelle l'Ingrédient (D) est un correcteur de pH qui est un alcali organique sélectionné parmi la trométhamine, l'arginine, et un mélange de ceux-ci.

4. Composition selon l'une quelconque des revendications 1 à 3, dans laquelle l'Ingrédient (C) est un polymère soluble dans l'eau qui est un épaississant associatif de type uréthane sélectionné dans le groupe consistant en éther de bis-décyltétradéceth-20 de copolymère de PEG-240/HDI, copolymère de PEG-150/alcool stéarylique/SMDI, copolymère de PEG-150/alcool décylique/SMDI, polyuréthane-59 et un mélange de ceux-ci.

5. Composition selon l'une quelconque des revendications 1 à 4, dans laquelle la composition présente un pH de 5,5 à 7,5.

6. Composition selon l'une quelconque des revendications 1 à 5 comprenant
Ingrédient (A) : un phosphate d'adénosine et/ou un sel de celui-ci ;
Ingrédient (B) : un polymère réticulé d'acrylate d'alkyle en C10-30/acrylates ;
Ingrédient (D) : de la trométhamine et/ou de l'arginine ;
Ingrédient (E) : un composant huileux ; et
Ingrédient (F) : de l'eau,
dans laquelle la composition présente une viscosité, déterminée selon la méthode décrite dans la description, de 20 000 à 40 000 mPa·s, et un pH de 6 à 7.

7. Composition selon l'une quelconque des revendications 1 à 6, comprenant
Ingrédient (A) : du phosphate d'adénosine et/ou un sel de celui-ci ;
Ingrédient (B) : un polymère réticulé d'acrylate d'alkyle en C10-30/acrylates ;
Ingrédient (C) : éther de bis-décyltétradéceth-20 de copolymère de PEG-240/HDI ;
Ingrédient (E) : un composant huileux ; et
Ingrédient (F) : de l'eau,
dans laquelle la composition présente une viscosité, déterminée selon la méthode décrite dans la description, de 20 000 à 40 000 mPa·s.

8. Composition selon l'une quelconque des revendications 1 à 7, dans laquelle la composition comprend
de 0,001 à 10 % en poids de l'Ingrédient (A),
de 0,05 à 5 % en poids de l'Ingrédient (B) et
de 1 à 60 % en poids de l'Ingrédient (E).

9. Composition selon l'une quelconque des revendications 1 à 8, dans laquelle la composition comprend de 0,01 à 20 % en poids de l'Ingrédient (C).

10. Composition selon l'une quelconque des revendications 1 à 9 comprenant
Ingrédient (A) : de 1,5 à 3,5 % en poids d'un phosphate d'adénosine et/ou d'un sel de celui-ci ;
Ingrédient (B) : de 0,4 à 0,8 % en poids d'un polymère réticulé d'acrylate d'alkyle en C10-30/acrylates ;
Ingrédient (D) : de la trométhamine et/ou de l'arginine ;
Ingrédient (E) : de 5 à 20 % en poids d'un composant huileux ; et
Ingrédient (F) : de l'eau,
dans laquelle la composition présente une viscosité, déterminée selon la méthode décrite dans la description, de 20 000 à 40 000 mPa·s, et un pH de 6 à 7.

11. Composition selon l'une quelconque des revendications 1 à 10 comprenant
Ingrédient (A) : de 1,5 à 3,5 % en poids d'un phosphate d'adénosine et/ou d'un sel de celui-ci ;
Ingrédient (B) : de 0,4 à 0,8 % en poids d'un polymère réticulé d'acrylate d'alkyle en C10-30/acrylates ;
Ingrédient (C) : de 0,3 à 0,8 % en poids d'éther de bis-décyltétradéceth-20 de copolymère de PEG-240/HDI ;
Ingrédient (E) : de 5 à 20 % en poids d'un composant huileux ; et
Ingrédient (F) : de l'eau,
dans laquelle la composition présente une viscosité, déterminée selon la méthode décrite dans la description, de 20 000 à 40 000 mPa·s.

12. Composition selon l'une quelconque des revendications 1 à 11, dans laquelle
la composition ne comprend pas de correcteur de pH qui est un composé alcalin inorganique, ou,
la composition comprend un correcteur de pH qui est un composé alcalin inorganique, et le correcteur de pH qui est un composé alcalin inorganique/Ingrédient (D) (rapport pondéral) est de 0,1 ou moins.

13. Composition selon l'une quelconque des revendications 1 à 12,
dans laquelle l'Ingrédient (E) comprend l'Ingrédient (G) sélectionné parmi :
une huile non cristallisée à point de fusion élevé présentant un point de fusion de 30 °C ou plus sélectionnée parmi un triglycéride non cristallisé présentant un point de fusion de 30 °C ou plus ; un ester d'acide gras de stérol non cristallisé présentant un point de fusion de 30 °C ou plus ; de la gelée de pétrole ; et un mélange de ceux-ci ;
un agent gélifiant huileux sélectionné parmi un ester d'acide gras d'inuline, un ester d'acide gras de dextrine, du polyglycérol, le dibutyléthylhexanoylglutamide, le dibutyllauroylglutamide, la lysine de dilauramidoglutamide sodique, un polymère réticulé de diméthicone de vinyle/silsesquioxane de méthicone, la diméthicone de PEG/PPG-19/19, et un mélange de ceux-ci ;
un agent silicone améliorant le toucher sélectionné parmi un silicone modifié par amino, une cire de silicone, un silicone modifié par polyéther, de la diméthicone hautement polymérisée, une cire de silicone, une émulsion de silicone, une poudre de silicone, et un mélange de ceux-ci ; et
un mélange de ceux-ci, et
dans laquelle la quantité de l'Ingrédient (G) est de 0,1 à 50 % en poids sur la base de l'Ingrédient (E).

14. Composition selon l'une quelconque des revendications 1 à 13 dans laquelle la composition comprend de 0,001 à 20 % en poids de l'Ingrédient (G).

15. Composition selon l'une quelconque des revendications 1 à 14, dans laquelle la composition comprend en outre
un Ingrédient (H) : un humectant hydrosoluble.
